# EUROPEAN PATENT SPECIFICATION

(11) **EP 1 392 270 B1**
(45) Date of publication and mention of the grant of the patent: **25.02.2009**
(21) Application number: 02736910.7
(22) Date of filing: 16.05.2002
(51) Int. Cl.: A61K 31/165, A61K 31/485

(54) **ABUSE RESISTANT PHARMACEUTICAL COMPOSITION CONTAINING CAPSAICIN**
CAPSAICIN-ENTHALTENDE MISSBRAUCHSICHERE PHARMAZEUTISCHE ZUSAMMENSETZUNGEN
COMPOSITION PHARMACEUTIQUE CONTENANT DE LA CAPSAICINE EMPECHANT LES ABUS

(30) Priority: 23.05.2001 US 292809 P
(43) Date of publication of application: 03.03.2004
(73) Proprietor: Endo Pharmaceuticals Inc., Chadds Ford, PA 19317 (US)
(72) Inventor: GOLDBERG, Michael, New York, NY 10128 (US); GALER, Bradley, Stuart, West Chester, PA 19382 (US); KAO, Huai-Hung, Syosset, NY 11791 (US)
(74) Representative: Buckley, Guy Julian
(86) International application number: PCT/US2002/015553
(87) International publication number: WO 2002/094254

(56) References cited:
- US-A- 4 599 342
- US-A- 4 681 897
- DATABASE BIOSIS [Online] BIOSCIENCES INFORMATION SERVICE, PHILADELPHIA, PA, US; 1994 BEHM ET AL.: "reducing craving for cigarettes while decreasing smoke intake using capsaicin-enhanced low tar cigarettes" Database accession no. 199598145755 XP002212281
- DATABASE MEDLINE [Online] US NATIONAL LIBRARY OF MEDICINE (NLM), BETHESDA, MD, US; 1996 RISTVEDT ET AL.: "the use of pharmacologic pain sensitization in the treatement of repetitive hair-pulling" Database accession no. nlm8870291 XP002212282

## Description

### Field of Invention

The invention relates to pharmaceutical compositions which include systems to deter abuse. More specifically, the invention relates to compositions containing an effective amount of pharmaceutical compound and capsaicin or a capsaicinoid compound. Most specifically, the invention relates to a composition containing an effective amount of a pharmaceutical compound, and an amount of a capsaicin compound to deter intranasal, oral, and intravenous abuse while having little or no irritating effect when administered orally or transdermally as directed.

### Description of the Related Art

The medicinal benefits of many pharmaceutical compounds, including narcotics and opioids, as well as non-narcotics and non-opioids, are well known and undisputed Unfortunately, the abuse and addiction to many of these drugs are equally undisputable. This is particularly true of opioids. Abusers, to gain the greatest effect, often snort powdered or dissolved versions of the drug or prepare a solution of the drug and inject it. Either way, the abuser can find a ready supply of addictive substances in prescription tablets and capsules, simply by crushing the tablets or capsules. Opioids are, perhaps, the most addictive and most abused of these compounds. Opioid as used herein means any opium product or analog thereof or other drug which acts on opioid receptors when intended for use as a pharmaceutical, including but not limited to codeine, dihydrocodeine, buprenorphine, fentanyl, hydrocodone, hydromorphone, levorphanol, meperidine, methadone, morphine, nalbuphine, oxycodone, oxymorphone, pentazocine, and propoxyphene, tramadol. In addition, other medications which have been abused include medication in the following drug classes: benzodiazepines, such as Valium, and NMDA-antagonists, such as ketamine.

With the advent of controlled release tablets and other formulations, ever increasing amounts of the effective pharmaceutical compounds are being formulated into a single tablet. Just as these formulations offer benefits to patients in increased effectiveness and convenience, each also provides greater and more convenient amounts of the abused drug to the addict. Accordingly, new and better ways to deter abuse have been sought. Because intranasal and intravenous abuse prove to be the most desirable and most dangerous methods of achieving a euphoric effect, i.e. "high", from these drugs, deterring such use would be beneficial. Deterring oral abuse would also be helpful.

Capsaicin, the natural ingredient found in chili peppers and other species of the Capsicium genus, is known to be an irritant. It is irritating, particularly to mucosal membranes, such as those found in the nasal passages. It has found recent fame for its use in "pepper spray", the layman's alternative to mace. In this situation, the pepper spray is effective to immobilize a would-be attacker for a period of time sufficient for escape. Capsaicin also has been used experimentally to study human neuropathic pain by injecting it into skin or muscle. It has been found that small amounts of capsaicin injected in this way causes pain which may last for hours. Other capsaicin analogues or capsaicinoids also appear to show similar effects. Throughout this specification and claims, the term "capsaicin" is used to denote capsaicin, and the term "capsaicinoid" is used to denote a broader class of compounds including capsaicin whether natural or synthetic, its analogs, and other derivatives and compounds generally referred to in the art as capsaicinoids.

Studies have suggested that capsaicin can be combined with various opioids with positive analgesic results. Capsaicin has not been found to act antagonistically or to interfere with the bioavailablity of the opioid. U. S. Patent No. 4,599,342, even suggests that capsaicin and opioids in certain ratios have a synergistic effect in producing analgesia. Ratios of capsaicin to opioid between 20,000:1 to 1:20 are shown in that patent. The '342 patent also shows that in oral dosages, the minimum effective dose of capsaicin is about 100mg for an average adult or about 1.3mg/kg, provided the capsaicin to opioid ratio is maintained. Doses range up to 2000 mg. U.S. Patent No. 4,681,897 suggests similar synergistic effects with non-opioid analgesics such as non-steroidal, analgesic/anti-inflammatory drugs (NSAIDs). The suggested minimum capsaicin content is 50mg (0.85 mg/kg) for an average adult, provided similar capsaicin to analgesic ratios are maintained. Acceptable doses here can be up to 2000 mg. However, in the '897 patent, the dosage forms are all for oral or transdermal use. Capsaicin has been shown to reduce craving for cigarettes (Behm et al., Exp. Clin. Psychopharmacology, 1994, 2(2), 143-153).

Interestingly, although capsaicin itself has been found to have analgesic properties, it still has been used as an irritant to trigger coughing, sneezing, and other ill effects in testing other analgesics. Capsaicin, therefore, has been used for seemingly opposite purposes. On one hand, it is an irritant, causing coughing, sneezing, pain and other effects, while on the other it has been purported to enhance analgesia in animal models of pain.

Because high potency and high content versions of abusive, but medicinally sanctioned, substances are becoming more readily available in solid oral dosage forms and transdermal formulations, new compositions and methods which allow effective medicinal use of the substance through an oral or transdermal route, while simultaneously deterring intranasal and/or intravenous abuse are needed. The present invention provides a solution to this problem.

The present invention is as set out in the accompanying claims.

### Summary of the Invention

A pharmaceutical composition intended for oral use contains the effective ingredient(s), capsaicin, and other materials used for dose formation. The composition is in the form of a solid oral dosage form, generally either a tablet or capsule. Aside from the effective pharmaceutical ingredient(s) the composition includes an amount of capsaicin which serves as a deterrent to the intranasal, oral or intravenous use of the composition. Such a composition deters abusers from crushing prescription pharmaceutical tablets for abusive snorting and/or injection. The capsaicin can be incorporated directly into the dosage form, or it can be sequestered to reduce or eliminate its release. Further, additional substances which enhance the effect of the capsaicin may be included in the tablet

### Detailed Description of the Preferred Embodiment

The inventive composition allows for medicinally effective oral dosing of potentially abusive substances while deterring their abuse intranasally, orally, or intravenously. Because the deterring ingredients are particularly irritating when snorted or injected, this invention naturally encompasses solid oral dosage forms such as pills, tablets, capsules, and the like. It also encompasses liquid-filled solid oral dosage forms, since they could be similarly abused. The invention centers on tablets and capsules because these dosage forms are intended to be dispensed to patients for later use. Therefore, these forms are more likely to be targets for abuse. Dosage forms designed to enter the system intravenously or intranasally should not be formulated according to the invention.

With that in mind, the composition is formulated for delivery as a solid oral dosage form. As is well known in the art, such dosage forms may contain a number of inactive ingredients such as fillers, excipients, and time release formulations (including sustained release, extended release, etc.). The composition and its ultimate dosage form may be formulated by any known technique, and is not meant to be limited to any particular formulation method or form.

The composition will also contain an effective amount of the pharmaceutical ingredient. The actual amount of the pharmaceutical ingredient will vary depending upon the nature of the ingredient, the strength of the tablet sought, the condition to be treated, and the size of the intended patient as well as many other factors. Larger doses and time released formulations have the potential to contain enough pharmaceutical ingredient for what traditionally would have been given in two or more doses throughout a day. Each of these tablets is likely to contain multiple potentially abusable doses of the active ingredient. Potentially abusable doses will vary in amount depending upon the active ingredient. Such amounts are readily ascertainable. These high content formulations are particularly susceptible to abuse; offering the abuser a concentrated source of opioid. In addition, some pharmaceutical analgesic drugs are composed of both an opioid and other analgesic drugs, such as acetaminophen, aspirin, and other non-steroidal anti-inflammatory drugs. The scope of the invention also includes these combination analgesic drugs.

Capsaicin is added such that when the tablet is crushed and taken intranasally by snorting, or intravenously by injection of a solution created from the crushed dose, severe irritating effects of the capsaicin are immediately felt. These irritating effects include, but are not limited to, coughing, sneezing, burning, and pain. The pain and discomfort associated with capsaicin may endure for minutes and potentially hours, and should deter subsequent or continued abuse. If the composition is crushed and snorted, pain and severe sneezing will result. Further, the sneezing induced by the capsaicin may cause the abuser to expel the opioid and thus help prevent abuse immediately. Capsaicin injected directly into a vein may not be painful, but should the abuser miss the vein by even a little, the pain from the resulting subcutaneous capsaicin will be excruciating. This should provide a deterring effect against future abuse. The tablet will also deter oral abuse. Oral abuse may occur in either of two ways. First, the abuser may simply chew the tablet. This breaks any controlled-release matrix of the tablet and releases all of the opioid immediately. This gives the abuser a strong euphoric feeling or "high." The other method for orally abusing an opioid tablet is by crushing and dissolving the tablet. The abuser then drinks the solution to get an immediate release of all of the opioid, again generating a "high." Either way, the inclusion of capsaicin according to the present invention can have a pungent taste, or may cause pain, when the tablet is crushed and dissolved, or chewed. In individuals sensitive to the effects of capsaicin, this helps deter future abuse due to the pungent taste or pain generated by the capsaicin.

The capsaicin can be added in either of two principal ways. First, the capsaicin can be incorporated directly into the matrix of the tablet. This will prevent abuse by crushing the tablet because such actions would release the capsaicin and cause severe discomfort to the potential abuser. Such a tablet would preferably be coated to delay release of capsaicin until after the tablet has reached the patient's stomach.

Alternatively, the capsaicin can be sequestered in the tablet so as to not release when the tablet is taken as intended. The preferred method of sequestering the capsaicin is by encapsulating it. Thus the tablet will comprise two separate matrices. The first, and generally more abundant, matrix will contain the tablet's active ingredient (i.e. opioid or other pharmaceutical). The second matrix will contain the capsaicin. The second matrix can be a homogenous controlled-release matrix (capsaicin in an ultra-slow release matrix capable of releasing less than 20% of the capsaicin in 12-24 hours). Alternatively, it can be a coating over an immediate release matrix, e.g. capsaicin in an immediate release matrix with a coating over the matrix which prevents release of the capsaicin unless the coating is compromised (by chewing or crushing the tablet). The "immediate release matrix" may include traditional formulation ingredients, or it may be purely capsaicin. Furthermore, the capsaicin, whether in this or another matrix, may be mixed with other substances, or chemically altered directly, so.as to make it more irritating or painful to an abuser, such as by improving solubility, or by some other method.

Although throughout the present specification the term "capsaicin" is used to refer to an abuse deterring substance, it should be noted that other abuse deterring compounds can also be used, especially where the compounds are encapsulated. Thus, it should be understood that abuse-deterring agents useful in the present invention include any capsaicinoids which are potentially noxious or irritating to mucus membranes, without causing lasting damage, including, but not limited to capsaicin and capsaicin derivatives of the general formula: 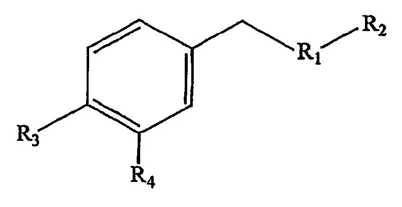
where R₁ is -NHC(O)-, -NHC(O)O-, -NHC(O)NH-, -NHC(S)NH-, -NHS(O₂)-, or-C(O)NH-;
R₂ is straight chain or branched C₅-C₁₁ alkyl, C₁₁-C₂₃ alkenyl, C₁₁-C₂₃ alkynyl, or C₁₁-C₂₃ alkadienyl;
R₃ is OH or a C₁-C₄ ester, and
R₄ is OH or OCH₃.

Particularly preferred capsaicin derivatives (also known as capsaicin analogs) are N-Vanillyl-9E-octadecenamide, 8-Methyl-N-vanillyl-6-nonenamide (capsaicin), dihydrocapsaicin, nordihydrocapcaisin, homocapsaicin, norcapsaicin, and nomorcapsaicin. Collectively and individually, these compounds (including capsaicin itself) are referred to herein as "capsaicimoids."

Of course it is most desirable to use those compounds most likely to cause physical discomfort without damage upon contact with mucous membranes. Those compounds would also be most likely to provide the sensation of heat when consumed. The sensation of heat provided by a compound can be determined the use of Scoville Units, a provided by a particular compound can be determined through the use of Scoville Units, a subjective scale of relative heat sensation loosely tied to the presence of capsaicinoids. A higher Scoville score indicates a higher degree of heat sensation. Of the capsaicinoids, those ranking highest on the Scoville scale are capsaicin, nordihydiocapsaicin, and dihydrocapsaicin, making these the most preferred compounds for use in the present invention. These compounds are shown below. Mixtures of these, and other capsaicinoids, are also included in the definition of capsaicin above and are useful in the present invention.

A minimal amount of capsaicin will produce the desired deterrent effects. This minimal amount should be included in every tablet regardless of tablet strength. Amounts below this minimum may not be effective to deter a determined addict Increased amounts of capsaicin should be included in a tablet containing a larger dose or time released doses to avoid dilution of the capsaicin, and, thus, its deterrent effects since these doses are more likely to be split by a potential abuser. Furthermore, in those situations where capsaicin is sequestered or encapsulated, additional capsaicin can be included since there is no chance of an adverse side effect where the capsaicin will not be released. However, the capsaicin should be maintained in a deterring range. Too much capsaicin should be avoided as it may provide an analgesic benefit which could overcome any deterring effect. Nevertheless, capsaicin content should be great enough to overcome any masking effects that may be imposed by the other ingredients in the tablet. Regardless of the situation, the intention is to provide a deterrent amount of capsaicin in the abusive dose that is derived from a tablet, to deter that abuse.

Because capsaicin is very irritating to the mucosal and vascular membranes, very small amounts of capsaicin will be effective. Just 75 micrograms has been shown to induce secretion, sneezing, and/or cough when introduced to the nasal mucosa of men and women. The minimum amount of capsaicin needed may be influenced by many factors, including the relative strength of the pharmaceutical ingredient, and the masking effect of other tablet components. Because some pharmaceutical agents are more likely to be abused by one particular route, the greatest potential route for abuse may also be taken into consideration when deciding how best to introduce the capsaicin into the tablet. Drugs abused intranasally may require a different amount of capsaicin than those which are abused intravenously or orally.

Table 1 illustrates preferred compositions containing the opioid, oxymorphone, and capsaicin in various tablet formulations. Other tablet ingredients are not included in the table. IR indicates immediate release formulation and ER indicates extended release formulation. In the case of oxymorphone, where a minimal effective dose is 2.5mg., less than 125 micrograms of capsaicin is preferred. This amount should be sufficient to deter abuse while being well within the range of oral and gastrointestinal acceptability. Amounts greater than this may be used, but amounts less than 125 micrograms should be sufficient to deter abuse.

**TABLE 1 Oxymorphone content (mg) and preferred Capsaicin content (mg)**

| | | | | | | |
|---|---|---|---|---|---|---|
| Oxymorphone IR | 2.5mg | 5mg | 10mg | | | |
| Capsaicin | <0.125mg | <0.25mg | <0.5mg | | | |
| Oxymorphone | | 5mg | 10mg | 20mg | 40mg | 80mg |
| ER Capsaicin | | <0.25mg | <0.5mg | <1.0mg | <2.0mg | <4.0mg |
| Oxycodone ER | | | 10mg | 20mg | 40mg | 80mg |
| Capsaicin | | | <0.5mg | <1.0mg | <2.0mg | <4.0mg |

As discussed above, considerations in establishing the amount of capsaicin include the amount of the pharmaceutical ingredient and its strength. Since oxymorphone is one of the stronger opioids, the preferred amount of less than 125 micrograms of capsaicin, should be effective in tablets containing other less powerful opioids or other drugs. More capsaicin, however, may be used especially where greater minimum dosage amounts are contemplated. For example, if a less potent pharmaceutical ingredient is present at 5mg, capsaicin is preferably present at less than 250 micrograms, and may be effective even at 125 micrograms or less. Further, more opioid in a tablet makes a tablet more likely to be abused, makes the use of the present invention more necessary.

As previously stated, it is the object of this invention to produce a tablet which, when abused, is painful to the abuser, and thus deters such abuse. The attempted abuse may be nasal (by snorting) or intravenous (by injection). Nasal discomfort should occur at a relatively low concentration due to the sensitivity of the nasal passages. However, the use of a capsaicinoid alone may cause a problem. That is, the opioid is generally water soluble, whereas capsaicin is not. Capsaicin and capsaicinoids tend to be hydrophobic. Therefore, the use of capsaicin alone can be ineffective against abuse by injection or by dissolution of the tablet followed by intranasal administration.

When a tablet containing an opioid and capsaicin is dissolved in water, the capsaicin will precipitate while the opioid dissolves. The liquid can then be drawn off and injected or snorted, leaving the capsaicin behind. This defeats the anti-abuse purpose of the capsaicin. This problem is solved by the addition of an emulsifying agent to the tablet. When the tablet is dissolved, the emulsifier allows the capsaicin to remain in the solution to be effective if snorted or injected. How much emulsifier is needed will depend on the emulsifier used and the particular capsaicinoid used.

However, for capsaicin and sodium lauryl sulfate, the following proportions have been determined to be effective.

| **Sodium Lauryl Sulfate (mg)** | **Capsaicin (mg)** |
|---|---|
| 0.56 | Negligible |
| 1.06 | 0.014 |
| 2.06 | 0.133 |
| 3.06 | 0.259 |
| 4.00 | 0.298 |
| 5.06 | 0.364 |

These proportions were determined by testing how much capsaicin would remain without precipitation in solutions having varying concentrations of sodium lauryl sulfate. The amounts of sodium lauryl sulfate shown are minimum amounts. If additional sodium lauryl sulfate is used, the invention will still operate. In practice, excess emulsifier should be used to insure there is sufficient emulsifier to emulsify all of the capsaicin. Again, a shortage of emulsifier will not cause the invention to be inoperable. Rather its effectiveness may be diminished.

Any suitable emulsifier can be used in the tablet of the present invention. Suitable emulsifiers include, but are not limited to, stearates such as sodium stearate, sorbitan monostearate and sorbitan tristearate, mono and diglycerides, laureates, oleates, glycols, or docusate sodium.

This preferred embodiment discusses the inventive composition in terms of capsaicin and oxymorphone, although other opioids and non-opioids may be used as the effective pharmaceutical ingredient. The invention, as described, is not limited to the specific compositions disclosed herein, but is useful with a variety of pharmaceutical compounds with potential for abuse.

As explained previously, the abuse-deterring agent (capsaicinoid) can be incorporated directly with the active pharmaceutical ingredient in pharmaceutically acceptable matrix, or the agent can be incorporated into a separate matrix or encapsulated. Where the abuse-deterring agent is incorporated in a separate matrix, the amount of agent can be increased considerably, again within the bounds of an abuse-deterring amount. It should be noted that the amount of abuse-deterring agent (capsaicinoid) which reaches the abuser can also be controlled by the amount of emulsifier in the tablet. By keeping the emulsifier at the correct level, the amount of abuse-deterring agent in the tablet can be increased, without getting too much agent in the abusers dose. This is because the excess capsaicinoid will precipitate when the tablet is dissolved.

The abuse-deterring agent can be incorporated into a matrix which will not release the agent unless the matrix is broken through crushing, or it can be formed into microcapsules which similarly will not release the agent unless crushed. Since the agent will not be released to a legitimate user, the amount of agent can be above the level which can be incorporated in a standard tablet

In this embodiment of the present invention, the capsaicinoid is contained in a separate matrix from the opioid. That separate matrix can be formed in many different ways. One appropriate configuration is a uniform controlled release matrix with the capsaicinoid dispersed therein. That controlled release matrix is formulated and granulated into very small granules. These granules are then incorporated into the main matrix of the tablet. In this way, the capsaicinoid is contained in a separate controlled release matrix which forms part of the entire tablet. Upon ingestion, the principle matrix of the tablets, which contains the opioid, dissolves, releasing the opioid and also releasing the granules containing the capsaicinoid in a solid reduced release or non-release matrix. The granules would then pass through and out of the body, releasing only minimal capsaicinoid, or no capsaicinoid at all.

Another possible configuration for the tablet of the present invention is to incorporate the capsaicinoid into an immediate release matrix. The matrix is granulated and coated with a non-release coating, such as an acrylic polymer. The granules are incorporated into either an immediate release or a controlled release opioid tablet. Upon administration, the tablet releases opioid at the predetermined rate, but the coated granules release no capsaicinoid. Rather, the granules pass through the intestines and are eliminated from the patient. In this way, the coated granules act as an excipient and, under normal circumstances, have no pharmacological effect whatsoever. Any suitable controlled or immediate release matrix can be used for the capsaicinoid provided that the proper non-release coating is used along with it.

Alternatively, a reduced release rate granule can be formed using an immediate release matrix with a reduced release rate coating over the formed granules. Although the description of the invention describes a "non-release" matrix in one embodiment, it is possible that some leakage of capsaicinoid may occur where "non-release" is specified. Thus, in the definition of "non-release" as used herein should be included any reduced release matrix which allows less than 20 percent of the capsaicinoid to be released over a 12-hour period under normal conditions of oral administration. Of course, none of the "non-release" matrices described herein are intended to fully encapsulate the capsaicinoid so as to prevent release when the tablet is crushed or dissolved Furthermore, a suitable non-release coating can be formed by using several known coatings together on a granulated matrix-containing capsaicinoid. For instance, the capsaicinoid granules can be covered with a coating which allows for release of material only at a pH below 5 (or 3), which is then covered by a coating which allows release of material only at above a pH of 5 (or 7 or even 9). In that way, when the tablet is ingested, the outer coating will prevent release of material while the granules reside in the stomach, and the inner coating will prevent release of material once the tablet has passed through the stomach into the intestines, where the pH rises sufficiently to dissolve the outer coating. One skilled in the art would be able to formulate a suitable matrix for use in the tablet of the present invention.

The capsaicinoid need not be fully encapsulated so as to be inert. It may be desirable to allow some release of the capsaicinoid if small amounts will enhance the opioid's effectiveness through a synergistic effect. Thus, the encapsulation can provide variable release of the capsaicinoid depending on the formulation.

It is most preferable to use the tablet of the present invention with opioids having a high potential for abuse. Opioid agonists used in the present invention are set forth above and can be any agonist in general use as an analgesic, preferably including morphine, oxycodone, hydrocodone, codeine, dihydrocodeine, hydromorphone, propoxyphene, methadone, and oxymorphone. Specifically, any addictive opioid in an oral tablet form is the target of the present invention. Most particularly, controlled release oxycodone has recently been the target of abuse and would therefore make a good candidate for use in the present invention. However, while controlled release tablets have been a particular recent problem, the tablet of the present invention may be used for immediate release tablets as well as those in a controlled release format, as explained above.

The tablet of the present invention is intended for use with abuseable pharmaceuticals, principally opioids. Other aspects of the tablet should remain the same as tablets presently produced. Further, as with prior art opioid tablets, the tablets of the present invention may be combination tablets, including other pharmaceutical agents such as acetaminophen, aspirin, naproxen sodium, ibuprofen, other steroidal and non-steroidal antiinflammatories, COX-2 inhibitors, gabapentin, pregabalin, or other similar agents.

## Claims

1. A solid oral dosage composition comprising: an effective amount of a pharmaceutically active ingredient; and a capsaicinoid in an amount effective to cause at least one response selected from coughing, sneezing, secretion, and pain when contacted with a mucosal or vascular membrane or skin or muscle without an analgesic effect which overcomes said at least one response.

2. The solid oral dosage composition of claim 1, wherein said pharmaceutically active ingredient is an opioid.

3. The composition of claim 2, wherein said opioid is selected from the group consisting of oxycodone, hydromorphone, and oxymorphone.

4. The composition of claim 3 wherein said opioid is present at 2.5mg and said capsaicin is present at less than 0.125mg.

5. The composition of claim 3 wherein said opioid is present at 5.0mg and said capsaicin is present at less than 0.250mg.

6. The composition of claim 3 wherein said opioid is present at 10mg and said capsaicin is present at less than 0.5mg.

7. The composition of claim 3 wherein said opioid is present at 20mg and said capsaicin is present at less than 1.0mg.

8. The composition of claim 3 wherein said opioid is present at 40mg and said capsaicin is present at less than 2.0g.

9. The composition of claim 3 wherein said oxymorphone is present at 80mg and said capsaicin is present at less than 4.0mg.

10. A solid oral dosage composition as claimed in claim 1, comprising: multiple effective doses of a pharmaceutically active ingredient; capsaicin in an amount sufficient to induce one of sneezing, coughing, secretion, and pain when contacted with mucosal or vascular membranes without an analgesic effect which overcomes said at least one response for each of said multiple effective doses of said pharmaceutically active ingredient.

11. The composition of claim 10 wherein said composition is a controlled release formulation.

12. The solid oral dosage composition of claim 10, wherein said pharmaceutically active ingredient is an opioid.

13. The composition of claim 10, wherein said opioid is oxymorphone.

14. The composition of claim 10 wherein said oxymorphone is present at 10mg and said capsaicin is present at less than0. 5mg.

15. The composition of claim 10 wherein said oxymorphone is present at 20mg and said capsaicin is present at less than 1.0mg.

16. The composition of claim 10 wherein said oxymorphone is present at 40mg and said capsaicin is present at less than 2.0mg.

17. The composition of claim 10 wherein said oxymorphone is present at 80mg and said capsaicin is present at less than 4.0mg.

18. The composition of Claim 1 or claim 10 wherein said capsaicin is incorporated directly into the matrix with said opioid.

19. The composition of Claim 1 or claim 10 wherein said capsaicin is incorporated into a second matrix, separate from said active ingredient matrix.

20. The composition of Claim 1 or claim 10 wherein said capsaicin is encapsulated in a material which does not normally release said capsaicin when said composition is administered orally.

21. The composition of Claim 20 wherein capsaicin is encapsulated in a material which releases no more than 20% in a 12 hour period when administered orally.

22. The solid oral dose composition of Claim 1 or claim 10 wherein said pharmaceutically active ingredient is selected from the group consisting of opioids, non-steroidal anti-inflammatory drugs (NSAIDs),COX-1 and COX-2 inhibitors, benzodiazepines, and NMDA-antagonists.

23. A solid oral dosage composition as claimed in claim 1, wherein said composition is in the form of a tablet, and wherein the capsaicinoid is sequestered in the tablet so as not to release when the tablet is taken as intended.

## Patentansprüche

1. Eine feste oral einzunehmende Zusammensetzung enthaltend: eine wirksame Menge eines pharmazeutisch aktiven Bestandteils und ein Capsaicinoid in ausreichender Menge, um bei Kontakt mit einer Magen- oder Gefäßwandung oder Haut oder Muskelgewebe wenigstens eine der Reaktionen Husten, Niesen, Sekretion und Schmerz auszulösen, ohne eine schmerzlindernde Wirkung, die die genannte Reaktion übersteigt.

2. Eine feste oral einzunehmende Zusammensetzung nach Anspruch 1, worin dieser pharmazeutisch wirksame Bestandteil ein Opioid ist.

3. Eine Zusammensetzung nach Anspruch 2, worin dieses Opioid aus einer Gruppe ausgewählt ist, bestehend aus Oxycodon, Hydromorphon und Oxymorphon.

4. Eine Zusammensetzung nach Anspruch 3, worin das Opioid in einer Menge von 2,5 mg und das Capsaicin in einer Menge von weniger als 0,125 mg enthalten ist.

5. Eine Zusammensetzung nach Anspruch 3, worin das Opioid in einer Menge von 5,0 mg und das Capsaicin in einer Menge von weniger als 0,250 mg enthalten ist.

6. Eine Zusammensetzung nach Anspruch 3, worin das Opioid in einer Menge von 10 mg und das Capsaicin in einer Menge von weniger als 0,5 mg enthalten ist.

7. Eine Zusammensetzung nach Anspruch 3, worin das Opioid in einer Menge von 20 mg und das Capsaicin in einer Menge von weniger als 1,0 mg enthalten ist.

8. Eine Zusammensetzung nach Anspruch 3, worin das Opioid in einer Menge von 40 mg und das Capsaicin in einer Menge von weniger als 2,0 mg enthalten ist.

9. Eine Zusammensetzung nach Anspruch 3, worin das Oxymorphon in einer Menge von 80 mg und das Capsaicin in einer Menge von weniger als 4,0 mg enthalten ist.

10. Eine feste oral einzunehmende Zusammensetzung nach Anspruch 1, enthaltend: vielfach wirksamen Dosen eines pharmazeutisch wirksamen Bestandteils; ein Capsaicin in ausreichender Menge, um bei Kontakt mit einer Magen- oder Gefäßwandung wenigstens eine der Reaktionen Niesen, Husten, Sekretion und Schmerz auszulösen, ohne eine schmerzlindernde Wirkung, welche die zumindest eine Reaktion einer jeden dieser vielfach wirksamen Dosen dieses pharmazeutisch wirksamen Bestandteils übersteigt.

11. Eine Zusammensetzung nach Anspruch 10, worin die Zusammensetzung eine Formulierung mit kontrollierter Freisetzung ist.

12. Eine feste oral einzunehmende Zusammensetzung nach Anspruch 10, worin der pharmazeutisch wirksame Bestandteil ein Opioid ist.

13. Eine Zusammensetzung nach Anspruch 10, worin das Opioid Oxymorphon ist.

14. Eine Zusammensetzung nach Anspruch 10, worin das Oxymorphon in einer Menge von 10 mg und das Capsaicin in einer Menge von weniger als 0,5 mg enthalten ist.

15. Eine Zusammensetzung nach Anspruch 10, worin das Oxymorphon in einer Menge von 20 mg und das Capsaicin in einer Menge von weniger als 1,0 mg enthalten ist.

16. Eine Zusammensetzung nach Anspruch 10, worin das Oxymorphon in einer Menge von 40 mg und das Capsaicin in einer Menge von weniger als 2,0 mg enthalten ist.

17. Eine Zusammensetzung nach Anspruch 10, worin das Oxymorphon in einer Menge von 80 mg und das Capsaicin in einer Menge von weniger als 4,0 mg enthalten ist.

18. Eine Zusammensetzung nach Anspruch 1 oder Anspruch 10, worin das Capsaicin mit dem Opioid in eine Matrix eingearbeitet ist.

19. Eine Zusammensetzung nach Anspruch 1 oder Anspruch 10, worin das Capsaicin in eine zweite von der Matrix mit dem aktiven Bestandteil getrennten Matrix eingearbeitet ist.

20. Eine Zusammensetzung nach Anspruch 1 oder Anspruch 10, worin das Capsaicin in ein Material eingekapselt ist, das normalerweise das Capsaicin nicht freisetzt, wenn die Zusammensetzung oral eingenommen wird.

21. Eine Zusammensetzung nach Anspruch 20, worin das Capsaicin in ein Material eingekapselt ist, das bei oraler Einnahme normalerweise nicht mehr als 20% in 12 Stunden freisetzt.

22. Eine feste oral einzunehmende Zusammensetzung nach Anspruch 1 oder Anspruch 10, worin der pharmazeutisch aktive Bestandteil aus einer Gruppe ausgewählt ist, die aus Opioiden, nichtsteroidalen Entzündungshemmern (NSAIDs), COX-1 and COX-2 Inhibitoren, Benzodiazepinen und NMDA-Antagonisten besteht.

23. Eine feste oral einzunehmende Zusammensetzung nach Anspruch 1, worin die Zusammensetzung in Form einer Tablette vorliegt und worin das Capsaicinoid so in die Tablette eingearbeitet ist, dass es nicht freigesetzt wird, wenn die Tablette wie vorgesehen eingenommen wird.

## Revendications

1. Composition solide pour administration orale comprenant : une quantité effective d'un ingrédient actif du point de vue pharmaceutique ; et un capsaïcinoïde en quantité effective pour obtenir au moins une réponse choisie parmi la toux, l'éternuement, la sécrétion et la douleur, lorsque mis en contact avec une muqueuse ou une membrane vasculaire, la peau ou un muscle sans effet analgésique primant sur ladite au moins une réponse.

2. Composition solide pour administration orale selon la revendication 1, dans laquelle ledit ingrédient actif du point de vue pharmaceutique est un opioïde.

3. Composition selon la revendication 2, dans laquelle ledit opioïde est choisi dans le groupe composé par l'oxycodone, l'hydromorphone et l'oxymorphone.

4. Composition selon la revendication 3, contenant 2,5 mg dudit opioïde et moins de 0,125 mg de ladite capsaïcine.

5. Composition selon la revendication 3, contenant 5,0 mg dudit opioïde et moins de 0,250 mg de ladite capsaïcine.

6. Composition selon la revendication 3, contenant 10 mg dudit opioïde et moins de 0,5 mg de ladite capsaïcine.

7. Composition selon la revendication 3, contenant 20 mg dudit opioide et moins de 1,0 mg de ladite capsaïcine.

8. Composition selon la revendication 3, contenant 40 mg dudit opioïde et moins de 2,0 mg de ladite capsaïcine.

9. Composition selon la revendication 3, contenant 80 mg de ladite oxymorphone et moins de 4,0 mg de ladite capsaïcine.

10. Composition solide pour administration orale comme revendiqué dans la revendication 1, comprenant : de multiples doses effectives d'un ingrédient actif du point de vue pharmaceutique ; de la capsaïcine en quantité suffisante pour obtenir au moins une réponse choisie parmi la toux, l'éternuement, la sécrétion et la douleur, lorsque mis en contact avec une muqueuse ou une membrane vasculaire, sans effet analgésique primant sur ladite au moins une réponse pour chacune desdites multiples doses effectives dudit ingrédient actif du point de vue pharmaceutique.

11. Composition selon la revendication 10, dans laquelle ladite composition est une formulation à libération contrôlée.

12. Composition solide pour administration orale selon la revendication 10, dans laquelle ledit ingrédient actif du point de vue pharmaceutique est un opioïde.

13. Composition selon la revendication 10, dans laquelle ledit opioïde est de l'oxymorphone.

14. Composition selon la revendication 10, contenant 10 mg de ladite oxymorphone et moins de 0,5 mg de ladite capsaïcine.

15. Composition selon la revendication 10, contenant 20 mg de ladite oxymorphone et moins de 1,0 mg de ladite capsaïcine.

16. Composition selon la revendication 10, contenant 40 mg de ladite oxymorphone et moins de 2,0 mg de ladite capsaïcine.

17. Composition selon la revendication 10, contenant 80 mg de ladite oxymorphone et moins de 4,0 mg de ladite capsaïcine.

18. Composition selon la revendication 1 ou selon la revendication 10, dans laquelle ladite capsaïcine est incorporée directement dans la matrice avec ledit opioïde.

19. Composition selon la revendication 1 ou selon la revendication 10, dans laquelle ladite capsaïcine est incorporée dans une seconde matrice, distincte de ladite matrice contenant l'ingrédient actif.

20. Composition selon la revendication 1 ou selon la revendication 10, dans laquelle ladite capsaïcine est encapsulée dans un matériau qui ne libère normalement pas ladite capsaïcine lorsque ladite composition est administrée par voie orale.

21. composition selon la revendication 20, dans laquelle la capsaïcine est encapsulée dans un matériau qui ne libère pas plus de 20% sur une période de 12 heures en cas d'administration par voie orale.

22. Composition solide pour administration orale selon la revendication 1 ou 10, dans laquelle ledit ingrédient actif du point de vue pharmaceutique est choisi dans le groupe constitué des opioïdes, des anti-inflammatoires non stéroïdes (NSAIDs), des inhibiteurs cox-1 ET cox-2 , des benzodiazépines et des antagonistes NMDA.

23. Composition solide pour administration orale selon la revendication 1, dans laquelle ladite composition est sous forme de comprimé, et dans laquelle le capsaïcinolde est emprisonné dans le comprimé de façon à ne pas être libéré lorsque le comprimé n'est pas administré comme prévu.
